(19) Europäisches Patentamt
European Patent Office
Office européen des brevets

(11) **EP 4 712 024 A1**

(12) **EUROPEAN PATENT APPLICATION**

(43) Date of publication:
**18.03.2026 Bulletin 2026/12**

(21) Application number: **25201271.1**

(22) Date of filing: **10.09.2025**

(51) International Patent Classification (IPC):
**G06T 7/33** (2017.01)     **A61B 8/00** (2006.01)
**G06T 7/00** (2017.01)     **G06T 7/62** (2017.01)

(52) Cooperative Patent Classification (CPC):
**G06T 7/33; A61B 8/5261; G06T 7/0012;**
**G06T 7/62;** G06T 2207/10068; G06T 2207/10072;
G06T 2207/10081; G06T 2207/10132;
G06T 2207/20084; G06T 2207/30021;
G06T 2207/30101; G06T 2207/30172;
G06T 2207/30241; G06T 2211/456

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **13.09.2024 US 202418884190
13.09.2024 EP 24465572**

(71) Applicant: **Siemens Healthineers AG
91301 Forchheim (DE)**

(72) Inventors:
• **GULSUN, Mehmet Akif
Princeton, NJ, 08540 (US)**
• **ITU, Lucian Mihai
500294 Brasov (RO)**
• **SHARMA, Puneet
Princeton Junction, NJ, 08550 (US)**
• **LADES, Felix
91052 Erlangen (DE)**

(74) Representative: **HKW Intellectual Property PartG
mbB
Theresienhöhe 12
80339 München (DE)**

(54) **REGISTRATION OF INTRAVASCULAR AND NON-INVASIVE VASCULAR IMAGES FOR TRAINING AI MODELS**

(57) Systems and methods for determining a trajectory of one or more intravascular images in one or more non-invasive vascular images are provided. 1) one or more intravascular images of a vessel of a patient and 2) one or more non-invasive vascular images of the vessel of the patient are received. A trajectory of the one or more intravascular images in the one or more non-invasive vascular images is determined. The trajectory is output.

FIG. 1

100

Receive 1) one or more intravascular images of a vessel of a patient and 2) one or more non-invasive vascular images of the vessel of the patient
102

Determine a trajectory of the one or more intravascular images in the one or more non-invasive vascular images
104

Output the trajectory
106

**Description**

TECHNICAL FIELD

The present invention relates generally to medical imaging analysis, and in particular to registration of intravascular and non-invasive vascular images for training AI/ML (artificial intelligence/machine learning) models for performing medical imaging analysis tasks.

BACKGROUND

Non-invasive vascular imaging provides for the acquisition of images of vessels of a patient from outside of the body of the patient, without the need for catheter insertion. One example of non-invasive vascular imaging is CTA (computed tomography angiography). CTA provides for comprehensive imaging of the coronary vessels for the assessment of coronary artery disease. Conventionally, CTA images are manually annotated to delineate lumen and plaque in the vessels for training and validating AI/ML models for performing various medical imaging analysis tasks. However, there is high inter-user variability in such manual delineation of lumen and plaques in CTA images due to limited resolution and imaging artifacts such as, e.g., motion and calcium blooming.

BRIEF SUMMARY OF THE INVENTION

In accordance with one or more embodiments, systems, methods, apparatuses, and computer-program products for determining a trajectory of one or more intravascular images in one or more non-invasive vascular images are provided. 1) one or more intravascular images of a vessel of a patient and 2) one or more non-invasive vascular images of the vessel of the patient are received. A trajectory of the one or more intravascular images in the one or more non-invasive vascular images is determined. The trajectory is output.

In one embodiment, a cost function is defined. The cost function comprises at least one of: 1) a fit function that measures a similarity between a contour of the vessel in the one or more intravascular images and in the one or more non-invasive vascular images or 2) a fit function that measures a similarity between the one or more intravascular images and the one or more non-invasive vascular images. The cost function is optimized to determine the trajectory. In one embodiment, the cost function further comprises at least one of: a term of a distance between one or more landmarks in the one or more intravascular images and the one or more non-invasive vascular images; a term of a distance between consecutive points along the trajectory; a term of a curvature along the trajectory; or a term of a similarity of rotation angles between consecutive points along the trajectory.

In one embodiment, a unit tangent vector is predicted for each of the one or more intravascular images using a machine learning based model. The trajectory is constructed using the predicted unit tangent vectors based on one or more landmarks in the one or more intravascular images and the one or more non-invasive vascular images. The one or more intravascular images and the one or more non-invasive vascular images are registered using the trajectory by optimizing rotation angles based on at least one of: 1) a fit function that measures a similarity between a contour of the vessel in the one or more intravascular images and the one or more non-invasive vascular images or 2) a fit function that measures a similarity between the one or more intravascular images and the one or more non-invasive vascular images.

In one embodiment, a pullback of an imaging device acquiring the one or more intravascular images is simulated as the trajectory. The one or more intravascular images and the one or more non-invasive vascular images are registered based on the simulated pullback and one or more landmarks in the one or more intravascular images and the one or more non-invasive vascular images.

In one embodiment, a 3D mesh modelling the vessel is constructed based on contours in the one or more intravascular images using the trajectory.

In one embodiment, an angular registration between points on the trajectory and a centerline of the vessel is determined by optimizing rotation angles based on one or more of 1) a fit function that measures a similarity between a contour of the vessel in the one or more intravascular images and in the one or more non-invasive vascular images or 2) a fit function that measures a similarity between the one or more intravascular images and the one or more non-invasive vascular images.

In one embodiment, the one or more intravascular images comprise at least one of intravascular ultrasound images or optical coherence tomography images and the one or more non-invasive vascular images comprise computed tomography images.

These and other advantages of the invention will be apparent to those of ordinary skill in the art by reference to the following detailed description and the accompanying drawings.

BRIEF DESCRIPTION OF THE DRAWINGS

Figure 1 shows a method for determining a trajectory of one or more intravascular images in one or more non-invasive vascular images, in accordance with one or more embodiments;

Figure 2 shows a workflow for determining a trajectory of one or more intravascular images in one or more non-invasive vascular images, in accordance with one or more embodiments;

Figure 3 shows a workflow for determining a trajectory of one or more intravascular images using a machine learning based model, in accordance with one or more embodiments;

Figure 4 shows an exemplary artificial neural network that may be used to implement one or more embodiments;

Figure 5 shows a convolutional neural network that may be used to implement one or more embodiments;

Figure 6 shows a data flow diagram according to an embodiment for using a generative adversarial network that may be used to implement one or more embodiments;

Figure 7 shows a schematic structure of a recurrent machine learning model that may be used to implement one or more embodiments; and

Figure 8 shows a high-level block diagram of a computer that may be used to implement one or more embodiments.

DETAILED DESCRIPTION

The present invention generally relates to methods and systems for the registration of intravascular and non-invasive vascular images for training AI/ML models. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system. Further, reference herein to pixels of an image may refer equally to voxels of an image and vice versa.

Non-invasive vascular imaging (e.g., CTA) involves the acquisition of images of vessels of a patient from the outside of the body of the patient, without the need for catheter insertion. While CTA provides for comprehensive imaging of the vessels for the assessment of coronary artery disease, there is high inter-user variability in the manual delineation of lumen and plaques in CTA images due to limited resolution and imaging artifacts. Intravascular imaging involves the use of specialized catheters equipped with imaging devices to acquire high-resolution images of the interior of the vessels. Two techniques for acquiring intravascular imaging include IVUS (intravascular ultrasound) and OCT (optical coherence tomography). IVUS and OCT are the gold standard for delineating coronary lumen and plaques in blood vessels. Embodiments described herein provide for registration of intravascular images and non-invasive vascular images, thus enabling the lumen and plaques delineated in the intravascular images to be used as ground truths for training and validating AI/ML models for performing medical imaging analysis tasks.

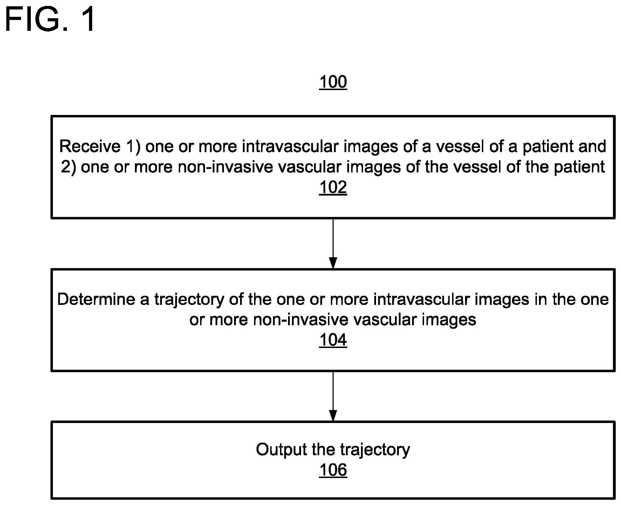

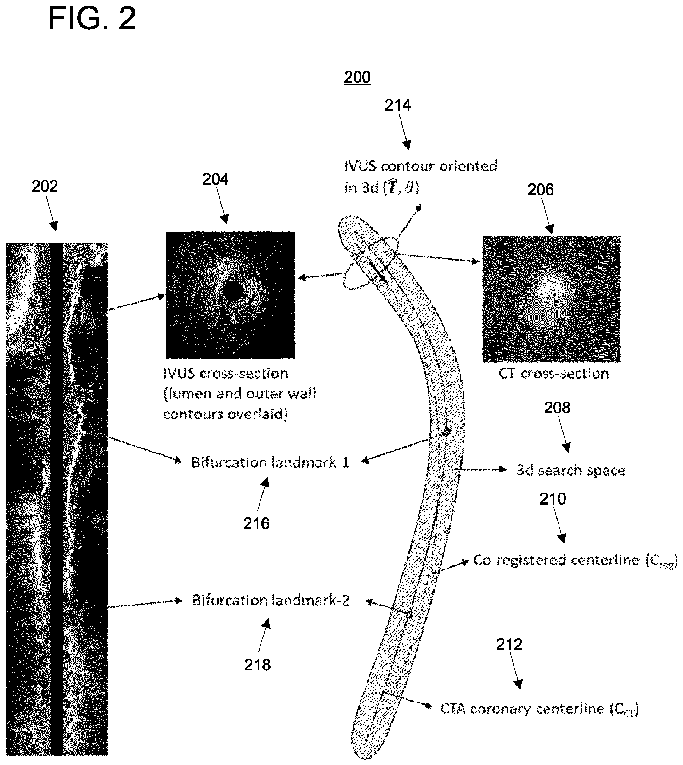

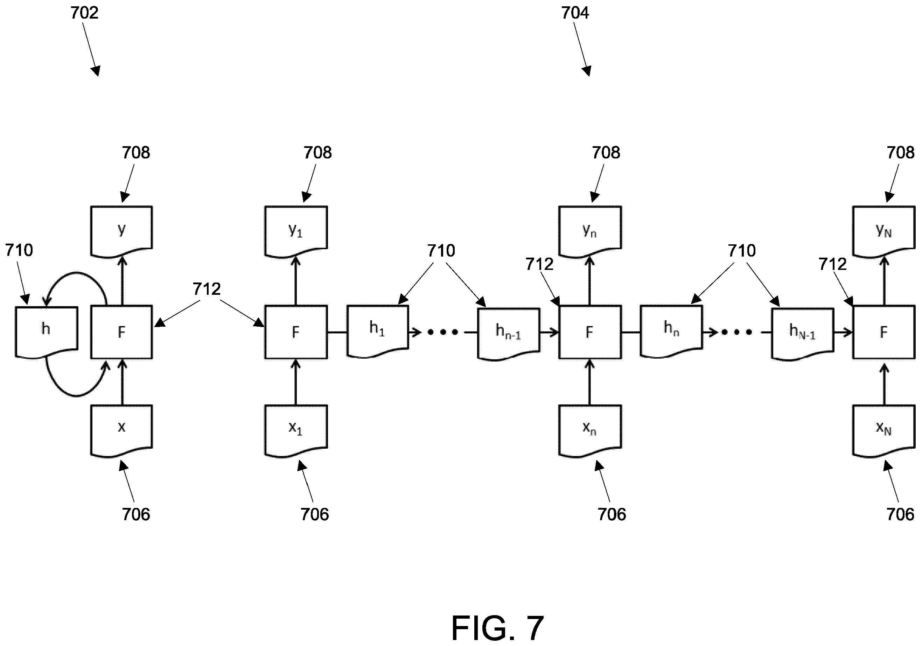

Figure 1 shows a method 100 for determining a trajectory of one or more intravascular images in one or more non-invasive vascular images, in accordance with one or more embodiments. The steps and sub-steps of method 100 may be performed by one or more suitable computing devices, such as, e.g., computer 702 of Figure 7. Figure 2 shows a workflow 200 for determining a trajectory of one or more intravascular images in one or more non-invasive vascular images, in accordance with one or more embodiments. Figure 1 and Figure 2 will be described together.

At step 102 of Figure 1, 1) one or more intravascular images of a vessel of a patient and 2) one or more non-invasive vascular images of the vessel of the patient are received. The one or more intravascular images and the one or more non-invasive vascular images are cross-sectional images of the patient depicting various features of the vessel, such as, e.g., lumen, vessel wall layers, plaques, stents, etc.

The one or more intravascular images of the vessel are images of the interior of the vessel invasively acquired, e.g., using a catheter equipped with an imaging device. In one embodiment, the one or more intravascular images comprise IVUS and OCT images. However, the one or more intravascular images may be of any other suitable modality or combination of modalities. The one or more intravascular images may comprise 2D (two dimensional) images and/or 3D (three dimensional) volumes, and may comprise a single image or a plurality images. To acquire the one or more intravascular images, a catheter equipped with an imaging device (e.g., ultrasound transducer or OCT imaging probe) is inserted into the vessel of the patient and the catheter is advanced to a region of interest within the vessel. The catheter is slowly pulled back through the vessel while the imaging probe captures images of the interior of the vessel to thereby acquire the one or more intravascular images. In one example, as shown in workflow 200 of Figure 2, the one or more intravascular images is IVUS image 204, of a sequence of IVUS images 202, showing a cross-section of the lumen and the outer wall of the vessel.

The one or more non-invasive vascular images of the vessel are images of the vessel acquired non-invasively or minimally invasively, e.g., without requiring the insertion of a catheter (or other medical instruction) into the patient. In one embodiment, the one or more non-invasive vascular images comprise CT (computed tomography) images (e.g., CTA). However, the one or more non-invasive vascular images may be of any other suitable modality or combination of modalities, such as, e.g., MRI (magnetic resonance imaging), PET (positron emission tomography), DSA (digital subtraction angiography), duplex ultrasound, etc. The one or more non-invasive vascular images may comprise 2D images and/or 3D volumes, and may comprise a single image or a plurality images. In one example, as shown in workflow 200 of Figure 2, the one or more non-invasive vascular images is CT image 206 showing a cross-section of the vessel.

The one or more intravascular images and one or more non-invasive vascular images may be received, for example, by directly receiving the images from the image acquisition devices (e.g., image acquisition device 714 of Figure 7) as the images are acquired, by loading the images from a storage or memory of a computer system (e.g., storage 712 or memory 710 of computer 702 of Figure 7), or by receiving the images from a remote computer system (e.g., computer 702 of Figure 7). Such a computer system or remote computer system may comprise one or more patient databases, such as, e.g. , an EHR (electronic health record), EMR (electronic medical record), PHR (personal health record), HIS (health information system), RIS (radiology information system), PACS (picture archiving and communication system), LIMS (laboratory information management system), or any other suitable database or system.

At step 104 of Figure 1, a trajectory of the one or more intravascular images is determined in the one or more non-invasive vascular images. As used herein, the trajectory of the one or more intravascular images refers to the path taken by an imaging device as the imaging device travels through the vessel to acquire the one or more intravascular images. In one example, as shown in workflow 200 of Figure 2, the trajectory is shown as a 3D co-registered centerline $C_{reg}$ 210. As the trajectory of the one or more intravascular images is determined in the one or more non-invasive vascular images, the trajectory represents the registration between the center point (or any other point defined to represent the trajectory) in the one or more intravascular images and the corresponding point on the trajectory in the one or more non-invasive vascular images, thereby registering the one or more intravascular images with the one or more non-invasive vascular images.

In one embodiment, the trajectory of the one or more intravascular images is determined by optimizing a cost function. For example, as shown in workflow 200 of Figure 2, the trajectory of the sequence of IVUS images 202 is determined by optimizing a cost function inside a 3D search space 208 defined around the centerline $C_{CT}$ 212 of the vessel determined from the CT image 206.

The cost function may be defined to include, for example, one or more of the following terms:

1) A fit function that measures a similarity between the cross-sectional contour 214 of the vessel in A) the one or more intravascular images (e.g., sequence of IVUS images 202) and B) the one or more non-invasive vascular images (e.g., CT image 206), on orthogonal planes corresponding to points along the trajectory. The contour 214 is oriented in 3D with a unit tangent $\hat{T}$ and rotation angle $\theta$ at each point on the trajectory. Unit tangent $\hat{T}$ is a vector that is tangent to the trajectory at a given point with a magnitude (length) of one. Unit tangent $\hat{T}$ provides a direction along the contour at the given point. Rotation angle $\theta$ is represented as a variable of the fit function (with a threshold on the rotation difference between consecutive points) and may be represented as a single rotation angle $\theta$ for all points on the trajectory or as different rotation angles $\theta$ for each point on the trajectory. An example similarity may be based on the fit of the contour in the one or more intravascular images to images features (e.g., edges and points of interest) in the one or more non-invasive vascular images.

2) A fit function that measures a similarity between A) the one or more intravascular images (e.g., sequence of IVUS images 202) and B) the one or more non-invasive vascular images (e.g., CT image 206), on orthogonal planes corresponding to points along the trajectory. An example fit function may be a mutual information-based intensity similarity measure.

3) A fit function that measures a similarity between plaques (e.g., calcified plaques) in A) the one or more intravascular images (e.g., sequence of IVUS images 202) and B) the one or more non-invasive vascular images (e.g., CT image 206), on orthogonal planes corresponding to points along the trajectory.

4) A term of a distance between one or more reference landmarks (such as, e.g., bifurcation landmarks 216 and 218, lesion markers, etc.) in the one or more intravascular images and the one or more non-invasive vascular images as a regularization term. The landmarks may be detected automatically, semi-automatically, and/or manually using, e.g., well-known methods.

5) A term of a distance between consecutive points along the trajectory as a regularization term.

6) A term of a curvature along the trajectory as a regularization term.

7) A term of a similarity of the rotation angle $\theta$ between consecutive points along the trajectory as a regularization term.

Once defined, the cost function is optimized within 3D search space 208 to determine the trajectory of the one or more intravascular images. For example, the cost function may be optimized as a continuous optimization program (e.g., using gradient descent, Newton's method, etc.) or via dynamic programming in a discrete space for efficiency. The trajectory represents the registration between the center point in the one or more intravascular images and the corresponding point on the trajectory in the one or more non-invasive vascular images.

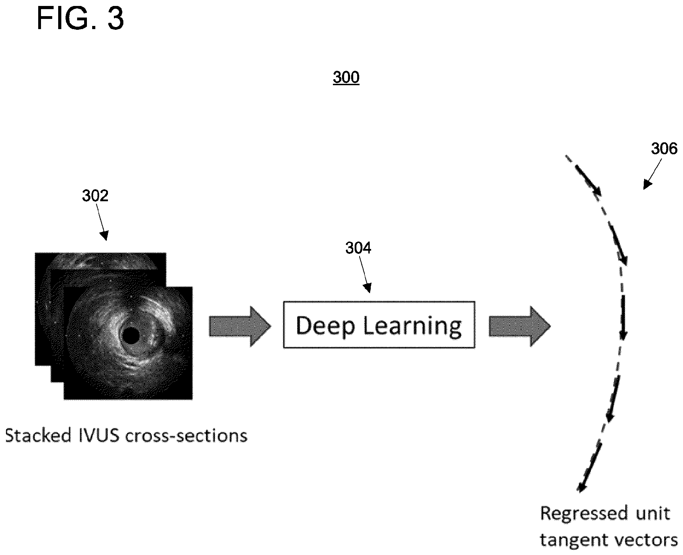

In one embodiment, the trajectory of the one or more intravascular images is determined using a machine learning based model to predict the unit tangent $\hat{T}$ for each of the one or more intravascular images. Figure 3 shows a workflow 300 for determining a trajectory of one or more intravascular images using a machine learning based model, in accordance with one or more embodiments. As shown in workflow 300, the machine learning based model is represented as deep learning model 304. Deep learning model 304 receives as input the one or more intravascular images (represented as stacked IVUS cross-sectional images 302) and generates as output a predicted unit tangent $\hat{T}$ 306 for each of the stacked IVUS cross-sectional images 302.

A 3D trajectory of the one or more intravascular images is constructed using the predicted unit tangents $\hat{T}$ 306 based on one or more reference landmarks defined in both the one or more intravascular images and the one or more non-invasive vascular images. In one embodiment, an angular registration between each point on the trajectory and the centerline of the vessel is also determined. The angular registration allows, for example, for validation or use as ground truth plaque information. The angular registration may be performed by optimizing the rotation angles $\theta$ based on one or more of 1) the fit function that measures a similarity between the cross-sectional contour of the vessel in A) the one or more intravascular images and B) the one or more non-invasive vascular images or 2) the fit function that measures a similarity between A) the one or more intravascular images and B) the one or more non-invasive vascular images, as described above.

Referring back to step 104 of Figure 1, in one embodiment, longitudinal images of the one or more intravascular images or the one or more non-invasive vascular images may be used as additional input to the machine learning based model. The machine learning based model is trained during a prior offline or training stage using manually registered pairs of intravascular images and non-invasive vascular images. Once trained, the machine learning based model may be applied during an online or inference stage, e.g., to predict the unit tangents $\hat{T}$.

In one embodiment, the trajectory of the one or more intravascular images is determined by simulating the pullback of the imaging device/catheter acquiring of the one or more intravascular images. The trajectory is simulated based on geometry of the vessel extracted from the one or more non-invasive vascular images. The simulated trajectory is projected in the one or more non-invasive vascular images with respect to the centerline of the vessel. In one embodiment, the one or more intravascular images may be associated with the simulated trajectory based on one or more reference landmarks (e.g., bifurcations or lesion markers) defined in both the one or more intravascular images and the one or more non-invasive vascular images.

In one embodiment, a 3D mesh modelling the vessel depicted in the one or more intravascular images may be constructed based on the contours in the one or more intravascular images using the determined trajectory. The 3D mesh represents a model of the contour of the vessel, such as, e.g., lumen, outer wall, etc.

At step 106 of Figure 1, the trajectory is output. For example, the trajectory can be output by displaying the trajectory on a display device of a computer system (e.g., I/O 708 of computer 702 of Figure 7), storing the trajectory on a memory or storage of a computer system (e.g., memory 710 or storage 712 of computer 702 of Figure 7), or by transmitting the trajectory to a remote computer system (e.g., computer 702 of Figure 7).

In one embodiment, a preprocessing step is performed (prior to step 102 of Figure 1) to identify suitable intravascular images received at step 102 of Figure 1. Intravascular images may have suboptimal image quality (e.g., due to blood artifacts) and thus only a subset of the cross-sectional intravascular images may have suitable image quality for registration with the one or more non-invasive images. Further, some of the intravascular images may depict the catheter and thus should be removed. The preprocessing step may be performed to automatically, semi-automatically, or manually identify intravascular images with suitable image quality of do not depict the catheter.

In one embodiment, to account for motion, only a subset of the intravascular images that match the cardiac phase of the non-invasive vascular images are received at step 102 of Figure 1 for image registration.

In one embodiment, the one or more intravascular images typically depict vessels of interest while the one or more non-invasive vascular images typically depict all (or at least more) coronary vessels of the patient. The vessel depicted in the one or more intravascular images that correspond to the vessel depicted in the one or more non-invasive vascular images may be automatically determined as part of the registration of method 100 of Figure 1. For instance, method 100 may be applied to attempt to register the one or more intravascular images with all available vessels in the one or more non-invasive vascular images and select the vessel with the highest registration score.

Embodiments described herein may be performed for the following exemplary applications.

In one embodiment, method 100 of Figure 1 may be performed to generate objective ground truth data from intravascular images for validating AI/ML models for, e.g., lumen and outer wall segmentation.

In one embodiment, AI/ML models may be trained using non-invasive vascular images (e.g., CTA images) based on registered ground truth in intravascular images (e.g., IVUS/OCT images) according to method 100 of Figure 1.

In one embodiment, findings in non-invasive vascular images (e.g., CTA images) may be fused on intravascular images (e.g., IVUS/OCT images) as complementary information according to method 100 of Figure 1. For example, heavily calcified regions may not be clearly seen on intravascular images due to acoustic shadowing but seen in non-invasive vascular images. Fused images may allow for a more informed decision in PCI (percutaneous coronary intervention) planning.

In one embodiment, findings in non-invasive vascular images (e.g., CTA images) may be fused with XA (x-ray angiography) images, which may be registered with the intravascular images (e.g., IVUS/OCT images), e.g., using well-known approaches. By registering the non-invasive vascular images with the intravascular images according to method 100 of Figure 1 during intervention, the registration between the non-invasive vascular images and the XA images is indirectly obtained, which allows for a comprehensive fusion of plaque information in the XA images. Fused images may allow for a more informed decision in PCI (percutaneous coronary intervention) planning.

In one embodiment, the acquisition of the one or more intravascular images may be performed post-PCI. The stent section of the vessel may be excluded from the registration process. Fused images according to method 100 of Figure 1 may be employed to generate a virtual post-PCI non-invasive vascular image, i.e., where the stenosis region is replaced virtually by the stent.

In one embodiment, the display and interactions of the cross-sectional and longitudinal view of the vessel in the one or more intravascular images and the one or more non-invasive vascular images are synchronized based on the registration according to method 100 of Figure 1. The interactions may include, for example, scrolling along the centerline of the vessel, rotating CPR (curved planar reformation), zooming and panning, etc.

In one embodiment, a 3D mesh may be generated from the intravascular images based on the trajectory by interpolating the contours in the 3D patient space. The 3D mesh models the vessel to allow for more precise 3D measurements, such as, e.g., calculation of the 3D volume from the one or more intravascular images instead of applying Simpsons Rule integration of area curves.

In one embodiment, a GAN (generative adversarial network) may be used to generate one or more synthetic intravascular images from the one or more non-invasive vascular images, for example, using the cycleGAN approach. The one or more synthetic intravascular images may then be registered with the one or more (real) intravascular images. Similarly, a GAN may be used to generate one or more synthetic non-invasive vascular images from the one or more intravascular images and the one or more synthetic non-invasive vascular images may then be registered with the one or more (real) non-invasive vascular images.

In one embodiment, a GAN may be used to generate one or more synthetic intravascular images from the one or more non-invasive vascular images, for example, using the cycleGAN approach. The one or more synthetic intravascular images, along with known ground truth from non-invasive data (e.g., unit tangent vectors), can be used to train/pre-train the machine learning model for predicting a unit tangent vector.

Embodiments described herein are described with respect to the claimed systems as well as with respect to the claimed methods. Features, advantages or alternative embodiments herein can be assigned to the other claimed objects and vice versa. In other words, claims and embodiments for the systems can be improved with features described or claimed in the context of the respective methods. In this case, the functional features of the method are implemented by physical units of the system.

Furthermore, certain embodiments described herein are described with respect to methods and systems utilizing trained machine learning models, as well as with respect to methods and systems for providing trained machine learning models. Features, advantages or alternative embodiments herein can be assigned to the other claimed objects and vice versa. In other words, claims and embodiments for providing trained machine learning models can be improved with features described or claimed in the context of utilizing trained machine learning models, and vice versa. In particular, datasets used in the methods and systems for utilizing trained machine learning models can have the same properties and features as the corresponding datasets used in the methods and systems for providing trained machine learning models, and the trained machine learning models provided by the respective methods and systems can be used in the methods and systems for utilizing the trained machine learning models.

In general, a trained machine learning model mimics cognitive functions that humans associate with other human minds. In particular, by training based on training data the machine learning model is able to adapt to new circumstances and to detect and extrapolate patterns. Another term for "trained machine learning model" is "trained function."

In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning models can be adapted iteratively by several steps of training. In particular, within the training a certain cost function can be minimized. In particular, within the training of a neural network the backpropagation algorithm can be used.

In particular, a machine learning model, such as, e.g., the machine learning based networks utilized at step 104 of Figure 1 and the deep learning model 304 of Figure 3, can comprise, for example, a neural network, a support vector machine, a decision tree and/or a Bayesian network, and/or the machine learning model can be based on, for example, k-means clustering, Q-learning, genetic algorithms and/or association rules. In particular, a neural network can be, e.g., a deep neural network, a convolutional neural network or a convolutional deep neural network. Furthermore, a neural network can be, e.g., an adversarial network, a deep adversarial network and/or a generative adversarial network.

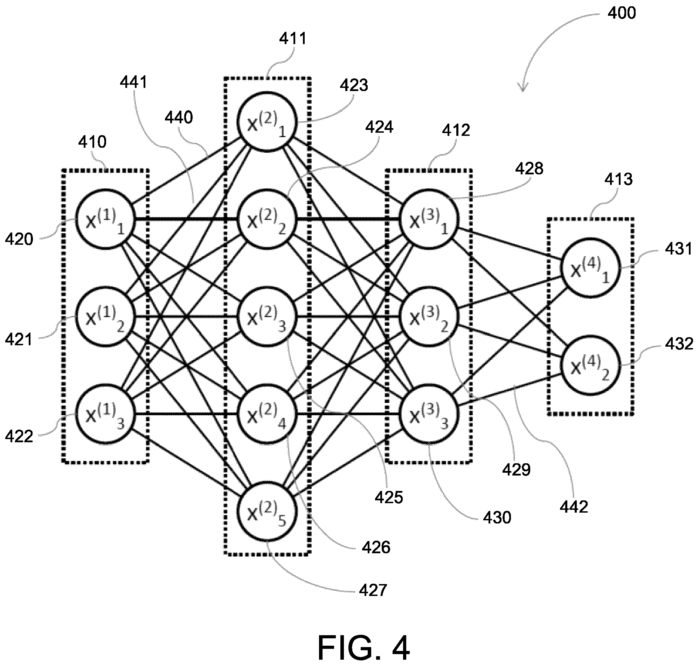

Figure 4 shows an embodiment of an artificial neural network 400 that may be used to implement one or more machine learning models described herein. Alternative terms for "artificial neural network" are "neural network", "artificial neural net" or "neural net".

The artificial neural network 400 comprises nodes 420, ..., 432 and edges 440, ..., 442, wherein each edge 440, ..., 442 is a directed connection from a first node 420, ..., 432 to a second node 420, ..., 432. In general, the first node 420, ..., 432 and the second node 420, ..., 432 are different nodes 420, ..., 432, it is also possible that the first node 420, ..., 432 and the second node 420, ..., 432 are identical. For example, in Figure 4 the edge 440 is a directed connection from the node 420 to the node 423, and the edge 442 is a directed connection from the node 430 to the node 432. An edge 440, ..., 442 from a first node 420, ..., 432 to a second node 420, ..., 432 is also denoted as "ingoing edge" for the second node 420, ..., 432 and as "outgoing edge" for the first node 420, ..., 432.

In this embodiment, the nodes 420, ..., 432 of the artificial neural network 400 can be arranged in layers 410, ..., 413, wherein the layers can comprise an intrinsic order introduced by the edges 440, ..., 442 between the nodes 420, ..., 432. In particular, edges 440, ..., 442 can exist only between neighboring layers of nodes. In the displayed embodiment, there is an input layer 410 comprising only nodes 420, ..., 422 without an incoming edge, an output layer 413 comprising only nodes 431, 432 without outgoing edges, and hidden layers 411, 412 in-between the input layer 410 and the output layer 413. In general, the number of hidden layers 411, 412 can be chosen arbitrarily. The number of nodes 420, ..., 422 within the input layer 410 usually relates to the number of input values of the neural network, and the number of nodes 431, 432 within the output layer 413 usually relates to the number of output values of the neural network.

In particular, a (real) number can be assigned as a value to every node 420, ..., 432 of the neural network 400. Here, $x^{(n)}_i$ denotes the value of the i-th node 420, ..., 432 of the n-th layer 410, ..., 413. The values of the nodes 420, ..., 422 of the input layer 410 are equivalent to the input values of the neural network 400, the values of the nodes 431, 432 of the output layer 413 are equivalent to the output value of the neural network 400. Furthermore, each edge 440, ..., 442 can comprise a weight being a real number, in particular, the weight is a real number within the interval [-1, 1] or within the interval [0, 1]. Here, $w^{(m,n)}_{i,j}$ denotes the weight of the edge between the i-th node 420, ..., 432 of the m-th layer 410, ..., 413 and the j-th node 420, ..., 432 of the n-th layer 410, ..., 413. Furthermore, the abbreviation $w^{(n)}_{i,j}$ is defined for the weight $w^{(n,n+1)}_{i,j}$.

In particular, to calculate the output values of the neural network 400, the input values are propagated through the neural network. In particular, the values of the nodes 420, ..., 432 of the (n+1)-th layer 410, ..., 413 can be calculated based on the values of the nodes 420, ..., 432 of the n-th layer 410, ..., 413 by

$$x^{(n+1)_j} = f\left(\sum_i x^{(n)_i} \cdot w^{(n)_{i,j}}\right).$$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 410 are given by the input of the neural network 400, wherein values of the first hid-den layer 411 can be calculated based on the values of the input layer 410 of the neural network, wherein values of the second hidden layer 412 can be calculated based in the values of the first hidden layer 411, etc.

In order to set the values $w^{(m,n)}_{i,j}$ for the edges, the neural network 400 has to be trained using training data. In particular, training data comprises training input data and training output data (denoted as $t_i$). For a training step, the neural network 400 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 400 (backpropagation algorithm). In particular, the weights are changed according to

$$w'^{(n)_{i,j}} = w^{(n)_{i,j}} - \gamma \cdot \delta^{(n)_j} \cdot x^{(n)_i}$$

wherein $\gamma$ is a learning rate, and the numbers $\delta^{(n)}_j$ can be recursively calculated as

$$\delta^{(n)j} = \left( \sum_k \delta^{(n+1)k} \cdot w^{(n+1)j,k} \right) \cdot f'\left( \sum_i x^{(n)i} \cdot w^{(n)i,j} \right)$$

based on $\delta^{(n+1)}_j$, if the (n+1)-th layer is not the output layer, and

$$\delta^{(n)j} = \left( x^{(n+1)j} - t^{(n+1)j} \right) \cdot f'\left( x^{(n)i} \cdot w^{(n)i,j} \right)$$

if the (n+1)-th layer is the output layer 413, wherein f is the first derivative of the activation function, and $t^{(n+1)}_j$ is the comparison training value for the j-th node of the output layer 413.

A convolutional neural network is a neural network that uses a convolution operation instead general matrix multiplication in at least one of its layers (so-called "convolutional layer"). In particular, a convolutional layer performs a dot product of one or more convolution kernels with the convolutional layer's input data / image, wherein the entries of the one or more convolution kernel are the parameters or weights that are adapted by training. In particular, one can use the Frobenius inner product and the ReLU activation function. A convolutional neural network can comprise additional layers, e.g., pooling layers, fully connected layers, and normalization layers.

By using convolutional neural networks input images can be processed in a very efficient way, because a convolution operation based on different kernels can extract various image features, so that by adapting the weights of the convolution kernel the relevant image features can be found during training. Furthermore, based on the weight-sharing in the convolutional kernels less parameters need to be trained, which prevents overfitting in the training phase and allows to have faster training or more layers in the network, improving the performance of the network.

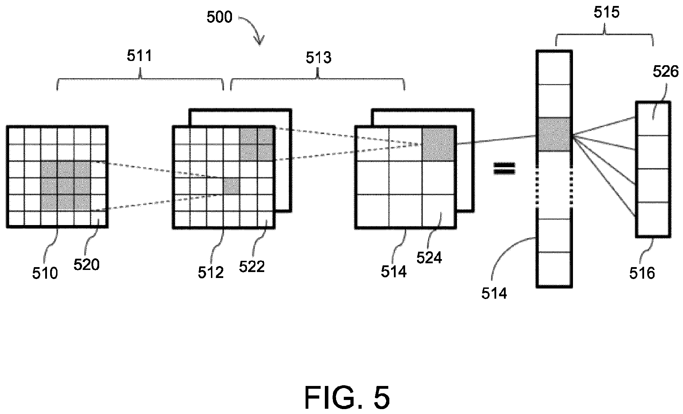

Figure 5 shows an embodiment of a convolutional neural network 500 that may be used to implement one or more machine learning models described herein. In the displayed embodiment, the convolutional neural network comprises 500 an input node layer 510, a convolutional layer 511, a pooling layer 513, a fully connected layer 514 and an output node layer 516, as well as hidden node layers 512, 514. Alternatively, the convolutional neural network 500 can comprise several convolutional layers 511, several pooling layers 513 and several fully connected layers 515, as well as other types of layers. The order of the layers can be chosen arbitrarily, usually fully connected layers 515 are used as the last layers before the output layer 516.

In particular, within a convolutional neural network 500 nodes 520, 522, 524 of a node layer 510, 512, 514 can be considered to be arranged as a d-dimensional matrix or as a d-dimensional image. In particular, in the two-dimensional case the value of the node 520, 522, 524 indexed with i and j in the n-th node layer 510, 512, 514 can be denoted as x(n)[i, j]. However, the arrangement of the nodes 520, 522, 524 of one node layer 510, 512, 514 does not have an effect on the calculations executed within the convolutional neural network 500 as such, since these are given solely by the structure and the weights of the edges.

A convolutional layer 511 is a connection layer between an anterior node layer 510 (with node values x(n-1)) and a posterior node layer 512 (with node values x(n)). In particular, a convolutional layer 511 is characterized by the structure and the weights of the incoming edges forming a convolution operation based on a certain number of kernels. In particular, the structure and the weights of the edges of the convolutional layer 511 are chosen such that the values x(n) of the nodes 522 of the posterior node layer 512 are calculated as a convolution x(n) = K * x(n-1) based on the values x(n-1) of the nodes 520 anterior node layer 510, where the convolution * is defined in the two-dimensional case as

$$x_k^{(n)}[i,j] = \left( K \ast x^{(n-1)} \right)[i,j] = \sum_{i'} \sum_{j'} K[i',j'] \cdot x^{(n-1)}[i-i', j-j'] .$$

Here the kernel K is a d-dimensional matrix (in this embodiment, a two-dimensional matrix), which is usually small compared to the number of nodes 520, 522 (e.g., a 3x3 matrix, or a 5x5 matrix). In particular, this implies that the weights of the edges in the convolution layer 511 are not independent, but chosen such that they produce said convolution equation. In particular, for a kernel being a 3x3 matrix, there are only 9 independent weights (each entry of the kernel matrix corresponding to one independent weight), irrespectively of the number of nodes 520, 522 in the anterior node layer 510 and the posterior node layer 512.

In general, convolutional neural networks 500 use node layers 510, 512, 514 with a plurality of channels, in particular, due to the use of a plurality of kernels in convolutional layers 511. In those cases, the node layers can be considered as (d+1)-dimensional matrices (the first dimension indexing the channels). The action of a convolutional layer 511 is then a two-dimensional example defined as

$$x^{(n)_b}[i,j] = \sum_a K_{a,b} * x^{(n\cdot 1)_a}[i,j] = \sum_a \sum_{i'} \sum_{j'} K_{a,b}[i',j'] \cdot x^{(n-1)_a}[i\cdot i', j\cdot j']$$

where $x^{(n-1)_a}$ corresponds to the a-th channel of the anterior node layer 510, $x^{(n)_b}$ corresponds to the b-th channel of the posterior node layer 512 and $K_{a,b}$ corresponds to one of the kernels. If a convolutional layer 511 acts on an anterior node layer 510 with A channels and outputs a posterior node layer 512 with B channels, there are $A \cdot B$ independent d-dimensional kernels $K_{a,b}$.

In general, in convolutional neural networks 500 activation functions are used. In this embodiment re ReLU (acronym for "Rectified Linear Units") is used, with R(z) = max(0, z), so that the action of the convolutional layer 511 in the two-dimensional example is

$$x^{(n)_b}[i,j] = R\left(\sum_a (K_{a,b} * x^{(n-1)_a})[i,j]\right) = R\left(\sum_a \sum_{i'} \sum_{j'} K_{a,b}[i',j'] \cdot x^{(n-1)_a}[i-i',j-j']\right)$$

It is also possible to use other activation functions, e.g., ELU (acronym for "Exponential Linear Unit"), LeakyReLU, Sigmoid, Tanh or Softmax.

In the displayed embodiment, the input layer 510 comprises 36 nodes 520, arranged as a two-dimensional 6x6 matrix. The first hidden node layer 512 comprises 72 nodes 522, arranged as two two-dimensional 6x6 matrices, each of the two matrices being the result of a convolution of the values of the input layer with a 3x3 kernel within the convolutional layer 511. Equivalently, the nodes 522 of the first hidden node layer 512 can be interpreted as arranged as a three-dimensional 2x6x6 matrix, wherein the first dimension correspond to the channel dimension.

The advantage of using convolutional layers 511 is that spatially local correlation of the input data can exploited by enforcing a local connectivity pattern between nodes of adjacent layers, in particular by each node being connected to only a small region of the nodes of the preceding layer.

A pooling layer 513 is a connection layer between an anterior node layer 512 (with node values x(n-1)) and a posterior node layer 514 (with node values x(n)). In particular, a pooling layer 513 can be characterized by the structure and the weights of the edges and the activation function forming a pooling operation based on a nonlinear pooling function f. For example, in the two-dimensional case the values x(n) of the nodes 524 of the posterior node layer 514 can be calculated based on the values x(n-1) of the nodes 522 of the anterior node layer 512 as

$$x^{(n)_b}[i,j] = f(x^{(n\cdot 1)}[id_1, jd_2], ..., x^{(n-1)_b}[(i+1)d_1 \cdot 1, (j+1)d_2 \cdot 1])$$

In other words, by using a pooling layer 513 the number of nodes 522, 524 can be reduced, by re-placing a number $d_1 \cdot d_2$ of neighboring nodes 522 in the anterior node layer 512 with a single node 522 in the posterior node layer 514 being calculated as a function of the values of said number of neighboring nodes. In particular, the pooling function f can be the max-function, the average or the L2-Norm. In particular, for a pooling layer 513 the weights of the incoming edges are fixed and are not modified by training.

The advantage of using a pooling layer 513 is that the number of nodes 522, 524 and the number of parameters is reduced. This leads to the amount of computation in the network being reduced and to a control of overfitting.

In the displayed embodiment, the pooling layer 513 is a max-pooling layer, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

In general, the last layers of a convolutional neural network 500 are fully connected layers 515. A fully connected layer 515 is a connection layer between an anterior node layer 514 and a posterior node layer 516. A fully connected layer 513 can be characterized by the fact that a majority, in particular, all edges between nodes 514 of the anterior node layer 514 and the nodes 516 of the posterior node layer are present, and wherein the weight of each of these edges can be adjusted individually.

In this embodiment, the nodes 524 of the anterior node layer 514 of the fully connected layer 515 are displayed both as two-dimensional matrices, and additionally as non-related nodes (indicated as a line of nodes, wherein the number of nodes was reduced for a better presentability). This operation is also denoted as "flattening". In this embodiment, the number of nodes 526 in the posterior node layer 516 of the fully connected layer 515 smaller than the number of nodes 524 in the anterior node layer 514. Alternatively, the number of nodes 526 can be equal or larger.

Furthermore, in this embodiment the Softmax activation function is used within the fully connected layer 515. By applying the Softmax function, the sum the values of all nodes 526 of the output layer 516 is 1, and all values of all nodes 526 of the output layer 516 are real numbers between 0 and 1. In particular, if using the convolutional neural network 500 for categorizing input data, the values of the output layer 516 can be interpreted as the probability of the input data falling into one of the different categories.

In particular, convolutional neural networks 500 can be trained based on the backpropagation algorithm. For preventing overfitting, methods of regularization can be used, e.g., dropout of nodes 520, ..., 524, stochastic pooling, use of artificial data, weight decay based on the L1 or the L2 norm, or max norm constraints.

According to an aspect, the machine learning model may comprise one or more residual networks (ResNet). In particular, a ResNet is an artificial neural network comprising at least one jump or skip connection used to jump over at least one layer of the artificial neural network. In particular, a ResNet may be a convolutional neural network comprising one or more skip connections respectively skipping one or more convolutional layers. According to some examples, the ResNets may be represented as m-layer ResNets, where m is the number of layers in the corresponding architecture and, according to some examples, may take values of 34, 50, 101, or 152. According to some examples, such an m-layer ResNet may respectively comprise (m-2)/2 skip connections.

A skip connection may be seen as a bypass which directly feeds the output of one preceding layer over one or more bypassed layers to a layer succeeding the one or more bypassed layers. Instead of having to directly fit a desired mapping, the bypassed layers would then have to fit a residual mapping "balancing" the directly fed output.

Fitting the residual mapping is computationally easier to optimize than the directed mapping. What is more, this alleviates the problem of vanishing/exploding gradients during optimization upon training the machine learning models: if a bypassed layer runs into such problems, its contribution may be skipped by regularization of the directly fed output. Using ResNets thus brings about the advantage that much deeper networks may be trained.

A generative adversarial model (an acronym is GA model) comprises a generative function and a discriminative function, wherein the generative function creates synthetic data, and the discriminative function distinguishes between synthetic and real data. By training the generative function and/or the discriminative function on the one hand the generative function is configured to create synthetic data which is incorrectly classified by the discriminative function as real, on the other hand the discriminative function is configured to distinguish between real data and synthetic data generated by the generative function. In the notion of game theory, a generative adversarial model can be interpreted as a zero-sum game. The training of the generative function and/or of the discriminative function is based, in particular, on the minimization of a cost function.

By using a GA model, based on a set of training data synthetic data can be generated that has the same characteristics as the training data set. The training of the GA model can be based on data not being annotated (unsupervised learning), so that there is low effort in training a GA model.

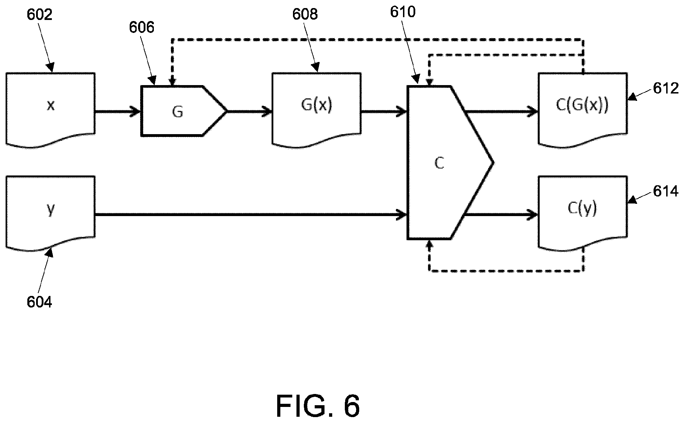

Figure 6 shows a data flow diagram according to an embodiment for using a generative adversarial network for creating synthetic output data G(x) 608 based on input data x 602 that is indistinguishable from real output data y 604, in accordance with one or more embodiments. The synthetic output data G(x) 608 has the same structure as the real output data y 604, but its content is not derived from real world data.

The generative adversarial network comprises a generator function G 606 and a classifier function C 610 which are trained jointly. The task of the generator function G 606 is to provide realistic synthetic output data G(x) 608 based on input data x 602, and the task of the classifier function C 610 is to distinguish between real output data y 604 and synthetic output data G(x) 608. In particular, the output of the classifier function C 610 is a real number between 0 and 1 corresponding to the probability of the input value being real data, so that an ideal classifier function would calculate an output value of C(y) 614 $\approx$ 1 for real data y 604 and C(G(x)) 612 $\approx$ 0 for synthetic data G(x) 608.

Within the training process, parameters of the generator function G 606 are adapted so that the synthetic output data G(x) 608 has the same characteristics as real output data y 604, so that the classifier function C 610 cannot distinguish between real and synthetic data anymore. At the same time, parameters of the classifier function C 610 are adapted so that it distinguishes between real and synthetic data in the best possible way. Here, the training relies on pairs comprising input data x 602 and the corresponding real output data y 604. Within a single training step, the generator function G 606 is applied to the input data x 602 for generating synthetic output data G(x) 608. Furthermore, the classifier function C 610 is applied to the real output data y 604 for generating a first classification result C(y) 614. Additionally, the classifier function C 610 is applied to the synthetic output data G(x) 608 for generating a second classification result C(G(x)) 612.

Adapting the parameters of the generative function G 606 and the classifier function C 610 is based on minimizing a cost function by using the backpropagation algorithm, respectively. In this embodiment, the cost function $K_C$ for the classifier function C 610 is $K_C \propto -BCE(C(y), 1) - BCE(C(G(x)), 0)$, wherein BCE denotes the binary cross entropy defined as $BCE(z, z') = z' \cdot log(z) + (1 - z') \cdot log(1 - z)$. By using this cost function, both wrongly classifying real output data as synthetic (indicated by $C(y) \approx 0$) and wrongly classifying synthetic output data as real (indicated as $C(G(x))$ 612 $\approx 1$) increases the cost function $K_C$ to be minimized. Furthermore, the cost function $K_G$ for the generator function G 606 is $K_G \propto -BCE(C(G(x)), 1) = -log(C(G(x))$. By using this cost function, correctly classified synthetic output data (indicated as $C(G(x))$ 612 $\approx 0$) leads to an increase of the cost function $K_G$ to be minimized.

In particular, a recurrent machine learning model is a machine learning model whose output does not only depend on the input value and the parameters of the machine learning model adapted by the training process, but also on a hidden state vector, wherein the hidden state vector is based on previous inputs used on for the recurrent machine learning model. In particular, the recurrent machine learning model can comprise additional storage states or additional structures that incorporate time delays or comprise feedback loops.

In particular, the underlying structure of a recurrent machine learning model can be a neural network, which can be denoted as recurrent neural network. Such a recurrent neural network can be described as an artificial neural network where connections between nodes form a directed graph along a temporal sequence. In particular, a recurrent neural network can be interpreted as directed acyclic graph. In particular, the recurrent neural network can be a finite impulse recurrent neural network or an infinite impulse recurrent neural network (wherein a finite impulse network can be unrolled and replaced with a strictly feedforward neural network, and an infinite impulse network cannot be unrolled and replaced with a strictly feedforward neural network).

In particular, training a recurrent neural network can be based on the BPTT algorithm (acronym for "backpropagation through time"), on the RTRL algorithm (acronym for "real-time recurrent learning") and/or on genetic algorithms.

By using a recurrent machine learning model input data comprising sequences of variable length can be used. In particular, this implies that the method cannot be used only for a fixed number of input datasets (and needs to be trained differently for every other number of input datasets used as input), but can be used for an arbitrary number of input datasets. This implies that the whole set of training data, independent of the number of input datasets contained in different sequences, can be used within the training, and that training data is not reduced to training data corresponding to a certain number of successive input datasets.

Figure 7 shows the schematic structure of a recurrent machine learning model F, both in a recurrent representation 702 and in an unfolded representation 704, that may be used to implement one or more machine learning models described herein. The recurrent machine learning model takes as input several input datasets x, $x_1$, ..., $x_N$ 706 and creates a corresponding set of output datasets y, $y_1$, ..., $y_N$ 708. Furthermore, the output depends on a so-called hidden vector h, $h_1$, ..., $h_N$ 710, which implicitly comprises information about input datasets previously used as input for the recurrent machine learning model F 712. By using these hidden vectors h, $h_1$, ..., $h_N$ 710, a sequentiality of the input datasets can be leveraged.

In a single step of the processing, the recurrent machine learning model F 712 takes as input the hidden vector $h_{n-1}$ created within the previous step and an input dataset $x_n$. Within this step, the recurrent machine learning model F generates as output an updated hidden vector $h_n$ and an output dataset $y_n$. In other words, one step of processing calculates $(y_n, h_n) = F(x_n, h_{n-1})$, or by splitting the recurrent machine learning model F 712 into a part F(y) calculating the output data and F(h) calculating the hidden vector, one step of processing calculates $y_n = F^{(y)}(x_n, h_{n-1})$ and $h_n = F^{(h)}(x_n, h_{n-1})$. For the first processing step, $h_0$ can be chosen randomly or filled with all entries being zero. The parameters of the recurrent machine learning model F 712 that were trained based on training datasets before do not change between the different processing steps.

In particular, the output data and the hidden vector of a processing step depend on all the previous input datasets used in the previous steps. $y_n = F^{(y)}(x_n, F^{(h)}(x_{n-1}, h_{n-2}))$ and $h_n = F(h)(x_n, F^{(h)}(X_{n-1}, h_{n-2}))$.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatuses, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatuses, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1-3. Certain steps or functions of the methods and workflows described herein, including one or more of the steps or functions of Figures 1-3, may be performed by a server or by another processor in a network-based cloud-computing system. Certain steps or functions of the methods and workflows described herein, including one or more of the steps of Figures 1-3, may be performed by a client computer in a network-based cloud computing system. The steps or functions of the methods and workflows described herein, including one or more of the steps of Figures 1-3, may be performed by a server and/or by a client computer in a network-based cloud computing system, in any combination.

Systems, apparatuses, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1-3, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

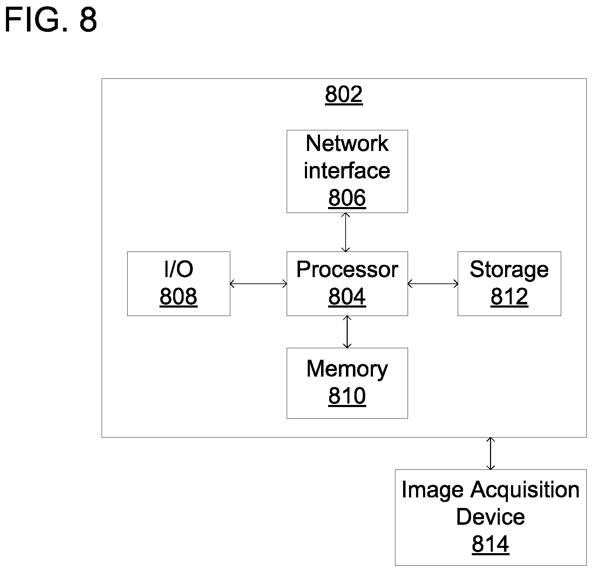

A high-level block diagram of an example computer 802 that may be used to implement systems, apparatuses, and methods described herein is depicted in Figure 8. Computer 802 includes a processor 804 operatively coupled to a data storage device 812 and a memory 810. Processor 804 controls the overall operation of computer 802 by executing computer program instructions that define such operations. The computer program instructions may be stored in data storage device 812, or other computer readable medium, and loaded into memory 810 when execution of the computer program instructions is desired. Thus, the method and workflow steps or functions of Figures 1-3 can be defined by the computer program instructions stored in memory 810 and/or data storage device 812 and controlled by processor 804 executing the computer program instructions. For example, the computer program instructions can be implemented as computer executable code programmed by one skilled in the art to perform the method and workflow steps or functions of Figures 1-3. Accordingly, by executing the computer program instructions, the processor 804 executes the method and workflow steps or functions of Figures 1-3. Computer 802 may also include one or more network interfaces 806 for communicating with other devices via a network. Computer 802 may also include one or more input/output devices 808 that enable user interaction with computer 802 (e.g., display, keyboard, mouse, speakers, buttons, etc.).

Processor 804 may include both general and special purpose microprocessors, and may be the sole processor or one of multiple processors of computer 802. Processor 804 may include one or more central processing units (CPUs), for example. Processor 804, data storage device 812, and/or memory 810 may include, be supplemented by, or incorporated in, one or more application-specific integrated circuits (ASICs) and/or one or more field programmable gate arrays (FPGAs).

Data storage device 812 and memory 810 each include a tangible non-transitory computer readable storage medium. Data storage device 812, and memory 810, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 808 may include peripherals, such as a printer, scanner, display screen, etc. For example, input/output devices 808 may include a display device such as a cathode ray tube (CRT) or liquid crystal display (LCD) monitor for displaying information to the user, a keyboard, and a pointing device such as a mouse or a trackball by which the user can provide input to computer 802.

An image acquisition device 814 can be connected to the computer 802 to input image data (e.g., medical images) to the computer 802. It is possible to implement the image acquisition device 814 and the computer 802 as one device. It is also possible that the image acquisition device 814 and the computer 802 communicate wirelessly through a network. In a possible embodiment, the computer 802 can be located remotely with respect to the image acquisition device 814.

Any or all of the systems, apparatuses, and methods discussed herein may be implemented using one or more computers such as computer 802.

One skilled in the art will recognize that an implementation of an actual computer or computer system may have other structures and may contain other components as well, and that Figure 8 is a high level representation of some of the components of such a computer for illustrative purposes.

Independent of the grammatical term usage, individuals with male, female or other gender identities are included within the term.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope of the invention. Those skilled in the art could implement various other feature combinations without departing from the scope of the invention as defined by the attached claims.

The following is a list of non-limiting illustrative embodiments disclosed herein:

Illustrative embodiment 1. A computer-implemented method comprising: receiving 1) one or more intravascular images of a vessel of a patient and 2) one or more non-invasive vascular images of the vessel of the patient; determining a trajectory of the one or more intravascular images in the one or more non-invasive vascular images; and outputting the trajectory.

Illustrative embodiment 2. The computer-implemented method of illustrative embodiment 1, wherein determining a trajectory of the one or more intravascular images in the one or more non-invasive vascular images comprises: defining a cost function comprising at least one of: 1) a fit function that measures a similarity between a contour of the vessel in the one or more intravascular images and in the one or more non-invasive vascular images or 2) a fit function that measures a similarity between the one or more intravascular images and the one or more non-invasive vascular images; and optimizing the cost function to determine the trajectory.

Illustrative embodiment 3. The computer-implemented method of illustrative embodiment 2, wherein the cost function further comprises at least one of: a term of a distance between one or more landmarks in the one or more intravascular images and the one or more non-invasive vascular images; a term of a distance between consecutive points along the trajectory; a term of a curvature along the trajectory; or a term of a similarity of rotation angles between consecutive points along the trajectory.

Illustrative embodiment 4. The computer-implemented method of any one of illustrative embodiments 1-3, wherein determining a trajectory of the one or more intravascular images in the one or more non-invasive vascular images comprises: predicting a unit tangent vector for each of the one or more intravascular images using a machine learning based model; and constructing the trajectory using the predicted unit tangent vectors based on one or more landmarks in the one or more intravascular images and the one or more non-invasive vascular images.

Illustrative embodiment 5. The computer-implemented method of illustrative embodiment 4, further comprising: registering the one or more intravascular images and the one or more non-invasive vascular images using the trajectory by optimizing rotation angles based on at least one of: 1) a fit function that measures a similarity between a contour of the vessel in the one or more intravascular images and the one or more non-invasive vascular images or 2) a fit function that measures a similarity between the one or more intravascular images and the one or more non-invasive vascular images.

Illustrative embodiment 6. The computer-implemented method of any one of illustrative embodiments 1-5, wherein determining a trajectory of the one or more intravascular images in the one or more non-invasive vascular images comprises: simulating a pullback of an imaging device acquiring the one or more intravascular images as the trajectory.

Illustrative embodiment 7. The computer-implemented method of illustrative embodiment 6, further comprising: registering the one or more intravascular images and the one or more non-invasive vascular images based on the simulated pullback and one or more landmarks in the one or more intravascular images and the one or more non-invasive vascular images.

Illustrative embodiment 8. The computer-implemented method of any one of illustrative embodiments 1-7, further comprising: constructing a 3D mesh modelling the vessel based on contours in the one or more intravascular images using the trajectory.

Illustrative embodiment 9. The computer-implemented method of any one of illustrative embodiments 1-8, further comprising: determining an angular registration between points on the trajectory and a centerline of the vessel by optimizing rotation angles based on one or more of 1) a fit function that measures a similarity between a contour of the vessel in the one or more intravascular images and in the one or more non-invasive vascular images or 2) a fit function that measures a similarity between the one or more intravascular images and the one or more non-invasive vascular images.

Illustrative embodiment 10. An apparatus comprising: means for receiving 1) one or more intravascular images of a vessel of a patient and 2) one or more non-invasive vascular images of the vessel of the patient; means for determining a trajectory of the one or more intravascular images in the one or more non-invasive vascular images; and means for outputting the trajectory.

Illustrative embodiment 11. The apparatus of illustrative embodiment 10, wherein the means for determining a trajectory of the one or more intravascular images in the one or more non-invasive vascular images comprises: means for defining a cost function comprising at least one of: 1) a fit function that measures a similarity between a contour of the vessel in the one or more intravascular images and in the one or more non-invasive vascular images or 2) a fit function that measures a similarity between the one or more intravascular images and the one or more non-invasive vascular images; and means for optimizing the cost function to determine the trajectory.

Illustrative embodiment 12. The apparatus of illustrative embodiment 11, wherein the cost function further comprises at least one of: a term of a distance between one or more landmarks in the one or more intravascular images and the one or more non-invasive vascular images; a term of a distance between consecutive points along the trajectory; a term of a curvature along the trajectory; or a term of a similarity of rotation angles between consecutive points along the trajectory.

Illustrative embodiment 13. The apparatus of any one of illustrative embodiments 10-12, wherein the means for determining a trajectory of the one or more intravascular images in the one or more non-invasive vascular images comprises: means for predicting a unit tangent vector for each of the one or more intravascular images using a machine learning based model; and means for constructing the trajectory using the predicted unit tangent vectors based on one or more landmarks in the one or more intravascular images and the one or more non-invasive vascular images.

Illustrative embodiment 14. The apparatus of illustrative embodiment 13, further comprising: means for registering the one or more intravascular images and the one or more non-invasive vascular images using the trajectory by optimizing rotation angles based on at least one of: 1) a fit function that measures a similarity between a contour of the vessel in the one or more intravascular images and the one or more non-invasive vascular images or 2) a fit function that measures a similarity between the one or more intravascular images and the one or more non-invasive vascular images.

Illustrative embodiment 15. A non-transitory computer-readable storage medium comprising instructions which, when executed by a computer, cause the computer to carry out operations comprising: receiving 1) one or more intravascular images of a vessel of a patient and 2) one or more non-invasive vascular images of the vessel of the patient; determining a trajectory of the one or more intravascular images in the one or more non-invasive vascular images; and outputting the trajectory.

Illustrative embodiment 16. The non-transitory computer-readable storage medium of illustrative embodiment 15, wherein determining a trajectory of the one or more intravascular images in the one or more non-invasive vascular images comprises: defining a cost function comprising at least one of: 1) a fit function that measures a similarity between a contour of the vessel in the one or more intravascular images and in the one or more non-invasive vascular images or 2) a fit function that measures a similarity between the one or more intravascular images and the one or more non-invasive vascular images; and optimizing the cost function to determine the trajectory.

Illustrative embodiment 17. The non-transitory computer-readable storage medium of any one of illustrative embodiments 15-16, wherein determining a trajectory of the one or more intravascular images in the one or more non-invasive vascular images comprises: simulating a pullback of an imaging device acquiring the one or more intravascular images as the trajectory.

Illustrative embodiment 18. The non-transitory computer-readable storage medium of illustrative embodiment 17, the operations further comprising: registering the one or more intravascular images and the one or more non-invasive vascular images based on the simulated pullback and one or more landmarks in the one or more intravascular images and the one or more non-invasive vascular images.

Illustrative embodiment 19. The non-transitory computer-readable storage medium of any one of illustrative embodiments 15-18, the operations further comprising: constructing a 3D mesh modelling the vessel based on contours in the one or more intravascular images using the trajectory.

Illustrative embodiment 20. The non-transitory computer-readable storage medium of any one of illustrative embodiments 15-19, the operations further comprising: determining an angular registration between points on the trajectory and a centerline of the vessel by optimizing rotation angles based on one or more of 1) a fit function that measures a similarity between a contour of the vessel in the one or more intravascular images and in the one or more non-invasive vascular images or 2) a fit function that measures a similarity between the one or more intravascular images and the one or more non-invasive vascular images.

**Claims**

1. A computer-implemented method comprising:

   receiving (102) 1) one or more intravascular images (204) of a vessel of a patient and 2) one or more non-invasive vascular images (206) of the vessel of the patient;
   determining (104) a trajectory (210) of the one or more intravascular images in the one or more non-invasive vascular images; and
outputting (106) the trajectory.

2. The computer-implemented method of claim 1, wherein determining a trajectory of the one or more intravascular images in the one or more non-invasive vascular images comprises:

defining a cost function comprising at least one of: 1) a fit function that measures a similarity between a contour (214) of the vessel in the one or more intravascular images and in the one or more non-invasive vascular images or 2) a fit function that measures a similarity between the one or more intravascular images and the one or more non-invasive vascular images; and
optimizing the cost function to determine the trajectory.

3. The computer-implemented method of claim 2, wherein the cost function further comprises at least one of:

a term of a distance between one or more landmarks (216, 218) in the one or more intravascular images and the one or more non-invasive vascular images;
a term of a distance between consecutive points along the trajectory;
a term of a curvature along the trajectory; or
a term of a similarity of rotation angles between consecutive points along the trajectory.

4. The computer-implemented method of any one of claims 1 - 3, wherein determining a trajectory of the one or more intravascular images in the one or more non-invasive vascular images comprises:

predicting a unit tangent vector for each of the one or more intravascular images using a machine learning based model; and
constructing the trajectory using the predicted unit tangent vectors based on one or more landmarks in the one or more intravascular images and the one or more non-invasive vascular images.

5. The computer-implemented method of claim 4, further comprising:
registering the one or more intravascular images and the one or more non-invasive vascular images using the trajectory by optimizing rotation angles based on at least one of: 1) a fit function that measures a similarity between a contour of the vessel in the one or more intravascular images and the one or more non-invasive vascular images or 2) a fit function that measures a similarity between the one or more intravascular images and the one or more non-invasive vascular images.

6. The computer-implemented method of any one of claims 1 - 5, wherein determining a trajectory of the one or more intravascular images in the one or more non-invasive vascular images comprises:
simulating a pullback of an imaging device acquiring the one or more intravascular images as the trajectory.

7. The computer-implemented method of claim 6, further comprising:
registering the one or more intravascular images and the one or more non-invasive vascular images based on the simulated pullback and one or more landmarks in the one or more intravascular images and the one or more non-invasive vascular images.

8. The computer-implemented method of any one of claims 1 - 7, further comprising:
constructing a 3D mesh modelling the vessel based on contours in the one or more intravascular images using the trajectory.

9. The computer-implemented method of any one of claims 1 - 8, further comprising:
determining an angular registration between points on the trajectory and a centerline of the vessel by optimizing rotation angles based on one or more of 1) a fit function that measures a similarity between a contour of the vessel in the one or more intravascular images and in the one or more non-invasive vascular images or 2) a fit function that measures a similarity between the one or more intravascular images and the one or more non-invasive vascular images.

10. An apparatus comprising:

means for receiving (102) 1) one or more intravascular images (204) of a vessel of a patient and 2) one or more non-invasive vascular images (206) of the vessel of the patient;
means for determining (104) a trajectory (210) of the one or more intravascular images in the one or more non-invasive vascular images; and
means for outputting (106) the trajectory.

11. The apparatus of claim 10, wherein the means for determining a trajectory of the one or more intravascular images in the one or more non-invasive vascular images comprises:

means for defining a cost function comprising at least one of: 1) a fit function that measures a similarity between a contour (214) of the vessel in the one or more intravascular images and in the one or more non-invasive vascular images or 2) a fit function that measures a similarity between the one or more intravascular images and the one or more non-invasive vascular images; and
means for optimizing the cost function to determine the trajectory.

12. The apparatus of claim 11, wherein the cost function further comprises at least one of:

a term of a distance between one or more landmarks (216, 218) in the one or more intravascular images and the one or more non-invasive vascular images;
a term of a distance between consecutive points along the trajectory;
a term of a curvature along the trajectory; or
a term of a similarity of rotation angles between consecutive points along the trajectory.

13. The apparatus of claim 10, wherein the means for determining a trajectory of the one or more intravascular images in the one or more non-invasive vascular images comprises:

means for predicting a unit tangent vector for each of the one or more intravascular images using a machine learning based model; and
means for constructing the trajectory using the predicted unit tangent vectors based on one or more landmarks in the one or more intravascular images and the one or more non-invasive vascular images.

14. The apparatus of claim 13, further comprising:
means for registering the one or more intravascular images and the one or more non-invasive vascular images using the trajectory by optimizing rotation angles based on at least one of: 1) a fit function that measures a similarity between a contour of the vessel in the one or more intravascular images and the one or more non-invasive vascular images or 2) a fit function that measures a similarity between the one or more intravascular images and the one or more non-invasive vascular images.

15. A non-transitory computer-readable storage medium comprising instructions which, when executed by a computer, cause the computer to carry out the method according to any one of claims 1 - 9.

# FIG. 1

<u>100</u>

Receive 1) one or more intravascular images of a vessel of a patient and 2) one or more non-invasive vascular images of the vessel of the patient
<u>102</u>

Determine a trajectory of the one or more intravascular images in the one or more non-invasive vascular images
<u>104</u>

Output the trajectory
<u>106</u>

# FIG. 2

# FIG. 3

<u>300</u>

302

304

306

Deep Learning

Stacked IVUS cross-sections

Regressed unit
tangent vectors

FIG. 4

FIG. 5

FIG. 6

FIG. 7

EP 4 712 024 A1

# FIG. 8

**802**

Network interface
**806**

I/O
**808**

Processor
**804**

Storage
**812**

Memory
**810**

Image Acquisition Device
**814**

Europäisches Patentamt

European Patent Office

Office européen des brevets

# EUROPEAN SEARCH REPORT

**Application Number**

EP 25 20 1271

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/334677 A1 (STURM BERNHARD [US]) 19 October 2023 (2023-10-19) | 1,6-10, 15 | INV. G06T7/33 |
| Y | * abstract, Figs. 1-20, [0001-0170], claims 1-19 * | 2,3,11, 12 | A61B8/00 G06T7/00 |
| A | | 4,5,13, 14 | G06T7/62 |
| | ----- | | |
| X | US 2015/131886 A1 (ABEN JEAN-PAUL [NL] ET AL) 14 May 2015 (2015-05-14) | 1,6-8, 10,15 | |
| Y | * abstract, Figs. 1-13, [0001-0083], | 2,11 | |
| A | claims 1-24 * | 4,5,13, 14 | |
| | ----- | | |
| X | US 2023/095242 A1 (LIU SHUBAO [US] ET AL) 30 March 2023 (2023-03-30) | 1-3,6-8, 10-12,15 | |
| A | * abstract, Figs. 1-15, [0001-0121], claims 1-20 * | 4,5,13, 14 | |
| | ----- | | |
| Y | HENK A. MARQUERING ET AL: "Coronary CT angiography: IVUS image fusion for quantitative plaque and stenosis analyses", PROCEEDINGS OF SPIE, VISUAL COMMUNICATIONS AND IMAGE PROCESSING 2005, vol. 6918, January 2008 (2008-01), page 69181G, XP055016049, Visual Communications and Image Processing 2005, 2005, Beijing, China ISSN: 0277-786X, DOI: 10.1117/12.772582 | 2,3,11, 12 | **TECHNICAL FIELDS SEARCHED (IPC)** G06T A61B |
| A | * abstract, Figs. 1-8, sections 1-4 * | 4,5,13, 14 | |
| | ----- | | |

The present search report has been drawn up for all claims

| Place of search | Date of completion of the search | Examiner |
|---|---|---|
| Munich | 26 January 2026 | Borotschnig, Hermann |

## ANNEX TO THE EUROPEAN SEARCH REPORT
## ON EUROPEAN PATENT APPLICATION NO.

EP 25 20 1271

This annex lists the patent family members relating to the patent documents cited in the above-mentioned European search report.
The members are as contained in the European Patent Office EDP file on
The European Patent Office is in no way liable for these particulars which are merely given for the purpose of information.

26-01-2026

| Patent document cited in search report | Publication date | Patent family member(s) | | Publication date |
|---|---|---|---|---|
| US 2023334677 A1 | 19-10-2023 | EP | 4222706 A2 | 09-08-2023 |
| | | US | 2023334677 A1 | 19-10-2023 |
| | | WO | 2022069327 A2 | 07-04-2022 |
| US 2015131886 A1 | 14-05-2015 | EP | 2873371 A1 | 20-05-2015 |
| | | JP | 6573447 B2 | 11-09-2019 |
| | | JP | 2015109968 A | 18-06-2015 |
| | | US | 2015131886 A1 | 14-05-2015 |
| US 2023095242 A1 | 30-03-2023 | NONE | | |

EPO FORM P0459

For more details about this annex : see Official Journal of the European Patent Office, No. 12/82